# EUROPEAN PATENT SPECIFICATION

(11) **EP 2 144 561 B1**
(45) Date of publication and mention of the grant of the patent: **20.09.2017**
(21) Application number: 08753830.2
(22) Date of filing: 16.05.2008
(51) Int. Cl.: A61B 5/055, G01N 24/08, G01N 33/483

(54) **MR METHODS OF GRADING A TUMOR USING AN IMAGING MEDIUM THAT COMPRISES HYPERPOLARISED 13C-PYRUVATE**
MRT-VERFAHREN ZUR GRADIERUNG EINES TUMORS MITHILFE EINES ABBILDUNGSMEDIUMS MIT HYDROPOLARISIERTEM 13C-PYRUVAT
PROCÉDÉS D'IMAGERIE PAR RÉSONANCE MAGNÉTIQUE D'ÉVALUATION D'UNE TUMEUR QUI COMPREND DU 13C-PYRUVATE HYPERPOLARISÉ

(30) Priority: 17.05.2007 US 938468 P
(43) Date of publication of application: 20.01.2010
(73) Proprietor: General Electric Company, Schenectady, NY 12345 (US); The Regents of the University of California, Oakland, CA 94607 (US)
(72) Inventor: HURD, Ralf, Eugene, Milpitas, California 95035 (US); KURHANEWICZ, John, South San Francisco, California 94080 (US); VIGNERON, Daniel, Blackburn, Corte Madera, California 94925 (US); NELSON, Sarah, Jane, Belmont, California 94002 (US)
(74) Representative: Livgard, Kim Are Birkeli
(86) International application number: PCT/NO2008/000171
(87) International publication number: WO 2008/143519

(56) References cited:
- WO-A-2006/011810
- HOWE F A; BARTON S J; CUDLIP S A; STUBBS M; SAUNDERS D E; MURPHY M;WILKINS P; OPSTAD K S; DOYLE V L; MCLEAN M A; BELL B A;: "Metabolic profiles of human brain tumors using quantitative in vivo 1H magnetic resonance spectroscopy" MAGNETIC RESONANCE IN MEDICINE, vol. 49, no. 2, February 2003 (2003-02), pages 223-232, XP002495766

## Description

### ABSTRACT

The invention relates to MR methods of grading a tumor using an imaging medium which comprises hyperpolarized ¹³C-pyruvate.

### BACKGROUND OF THE INVENTION

Prostate cancer is the most common type of cancer in men, excluding skin cancer, and is the second leading cause of cancer death. Risk factors include age; incidence increases in men over the age of 65 years. It is more prevalent in the western world and 10% of the cases can be linked to family history. Prostate cancer is a continuum, progressing through localized, locally advanced, advanced and hormone-refractory stages. In general it is a slow growing cancer which is typically under hormonal control, i.e. testosterone. Choice of treatment is dependent on the stage of disease and if detected early and treated appropriately, survival rates are excellent.

The presenting signs and symptoms of a prostate cancer vary as a function of its site of origin in the gland, and its extent of involvement. The majority of cancers arise in the peripheral zone and do not produce symptoms in the early stage of development. Those that arise in the transitional zone or that enlarge and encroach on the urethra, may produce hesitancy, a decrease in the force of urinary stream, intermittency and post-void leakage. However, all these symptoms may occur for other reasons, and there are no diagnostic symptoms or voiding pattern that can positively characterize prostate cancer. Due to improved public awareness and screening techniques, prostate cancer is being diagnosed in the earlier stages of development, often in men who are free of symptoms or asymptomatic.

A currently used screening procedure is PSA (prostate specific antigen) measurement. PSA is an enzyme secreted by the prostate gland and it is used as a marker for prostate disease. If not used for screening, PSA will usually be determined as part of the process of identifying someone likely to have prostate cancer. Especially tumors which are too small to be palpable on digital rectal examination (DRE), may only be suspected on PSA testing.

To confirm any findings of DRE/PSA, transrectal ultrasonography (TRUS) and TRUS guided needle biopsy are commonly used. However, especially for small localized tumors in the prostate the findings may be false-negative or false-positive: only 20-25% of cases of hypoechogenic images obtained with TRUS are confirmed to be prostate cancer. On the other hand, 25% of prostate tumors are isoechogenic. In biopsy usually 5 to 8 evenly spaced samples from different areas of the prostate are taken. In case of a small tumor, the biopsy needle may simply "miss" the tumor.

In WO-A-2006/011810 an *in vivo* MR imaging method was disclosed which allows for the discrimination between healthy and tumor tissue, especially prostate tumor tissue. An imaging medium comprising hyperpolarized ¹³C-pyruvate is used in the method.

Pyruvate is an endogenous compound which is very well tolerated by the human body, even in high concentrations. As a precursor in the citric acid cycle, pyruvate plays an important metabolic role in the human body. Pyruvate is converted into different compounds: its transamination results in alanine, via oxidative decarboxylation, pyruvate is converted into acetyl-CoA and bicarbonate, the reduction of pyruvate results in lactate and its carboxylation in oxaloacetate.

The metabolic conversion of hyperpolarized ¹³C-pyruvate to its metabolites hyperpolarized ¹³C-lactate, hyperpolarized ¹³C-bicarbonate (in the case of ¹³C₁-pyruvate, ¹³C_{1,2}-pyruvate or ¹³C_{1,2,3}-pyruvate only) and hyperpolarized ¹³C-alanine can be used for *in vivo* MR studying of metabolic processes in the human body. ¹³C₁-pyruvate has a T₁ relaxation in human full blood at 37°C of about 42 s, however, the conversion of hyperpolarized ¹³-pyruvate to hyperpolarized ¹³C-lactate, hyperpolarized ¹³C-bicarbonate and hyperpolarized ¹³C-alanine has been found to be fast enough to allow signal detection from the ¹³C-pyruvate parent compound and its metabolites. The amount of alanine, bicarbonate and lactate is dependent on the metabolic status of the tissue under investigation. The MR signal intensity of hyperpolarized ¹³C-lactate, hyperpolarized ¹³C-bicarbonate and hyperpolarized ¹³C-alanine is related to the amount of these compounds and the degree of polarization left at the time of detection, hence by monitoring the conversion of hyperpolarized ¹³C-pyruvate to hyperpolarized ¹³C-lactate, hyperpolarized ¹³C-bicarbonate and hyperpolarized ¹³C-alanine it is possible to study metabolic processes *in vivo* in the human or non-human animal body by using non-invasive MR imaging or MR spectroscopy.

It has been found that the MR signal amplitudes arising from the different ¹³C-pyruvate metabolites and thus the signal intensities vary depending on the tissue type. The unique MR metabolic pattern of signal intensities of alanine, lactate, bicarbonate and pyruvate can be used as fingerprint for the metabolic state of the tissue under examination and thus allows for the discrimination between healthy tissue and tumor tissue. Tumor tissue in ¹³C-MR images acquired of hyperpolarized ¹³C-pyruvate and its metabolites is indicated by the highest lactate signal or high weighted lactate over pyruvate or lactate over alanine signal, as described in detail in WO-A-2006/011810.

Magnetic resonance (MR) detection like for instance MR imaging (MRI) and MR spectroscopy (MRS) could be valuable tools for detecting prostate cancer and these tools have become particularly attractive to physicians as they allow for obtaining images of a patients body or parts thereof in a non-invasive way and without exposing the patient and the medical personnel to potentially harmful radiation such as X-ray. Because of its high quality images and good spatial and temporal resolution, MRI is the favorable imaging technique of soft tissue and organs.

It has now been found that *in vivo* MR imaging and/or MR spectroscopy of prostate cancer using an imaging medium that comprises hyperpolarized ¹³C-pyruvate may not only be used to discrimination between healthy and tumor tissue, but also for assessing prostate tumor aggressiveness, i.e. tumor grading.

Histological grading of prostate cancer is an important part of assessing the outcome, or prognosis, of the disease. Accurate grading of prostate cancer can help to predict the behavior and aggressiveness of the disease and the likely outcome for the patent.

Grading systems can assess the degree of cell anaplasia (variation in size, shape and staining properties) and differentiation, i.e. how well differentiated cells are, in the tumor. In the early stages of cancer, cells are usually well differentiated and function in a manner similar to the tissue from which they arise. As cancer progresses, the cells become less differentiated and begin to look and behave differently from their progenitors.

Various grading systems have become developed, but the one most widely used for prostate cancer is the Gleason system. The Gleason grading system is based on the extent to which the tumor cells are arranged into recognizably glandular structures and the level of cell differentiation. The Gleason system identifies five levels of increasing disease aggressiveness with grade 1 being the least aggressive and grade 5 being the most aggressive cancer.

Since most prostate cancers are heterogeneous, i.e. have a mixture of cells at different Gleason grades, the two most prominent grades are added together to produce the Gleason score. This score provides useful prognostic information and is, together with other parameters, used to make a decision on treatment of the prostate cancer. Gleason scores above 4 are associated with a risk of more rapid disease progression, increased potential for metastasis and decreased survival.

Although the Gleason grading is an attempt at objective quantification, histological grading, by its nature, is subjective. Specimens are prepared from cells derived from a biopsy sample of the tumor which are then microscopically examined by a skilled pathologist. A significant problem is the degree of interobserver and intraobserver variability in reading the same specimens. It may become necessary to obtain the opinion of several pathologists to reach a consensus on individual tumor grade. Also, Gleason grading requires samples of prostate tissue for analysis, which has to be obtained by (TRUS guided) biopsy. As mentioned earlier, results from a biopsy might be false-negative since especially in case of a small tumor, the biopsy needle may simply "miss" the tumor.

Hence there is a need for a method which is more consistently predicative of the aggressiveness of prostate cancer than the present subjective pathological analysis of biopsy sample of prostate tissue.

As described earlier, the metabolic state of the prostate tissue can be assessed by *in vivo* MR imaging of the prostate using an imaging medium that comprises hyperpolarized ¹³C-pyruvate. Tumor tissue is characterized by a higher metabolic activity than healthy tissue and an increased pyruvate to lactate conversion can be observed which results in a high ¹³C-lactate signal in such tumor tissue, making it possible to distinguish tumor tissue from healthy tissue and thus identifying tumor tissue.

### SUMMARY OF THE INVENTION

Surprisingly, we have now found that MR signals of hyperpolarized ¹³C-pyruvate and its metabolites can be used to assess prostate cancer aggressiveness and degree of malignancy and thus be used to grade prostate cancer. These findings are however not limited to prostate cancer but can be transferred to other cancer types. Thus by MR detection using an imaging medium comprising hyperpolarized ¹³C-pyruvarte, it is possible to not only identify tumor tissue but also make a prediction of the aggressiveness and degree of malignancy of said tumor, i.e. to grade the tumor using a completely non-invasive method. The invention is defined in the independent claim. We have found that early stage primary tumors (low grade tumors), late stage primary tumors (high grade tumors) and metastases do have characteristic metabolic profiles, i.e. a characteristic pattern of signals of ¹³C-pyruvate and its ¹³C-containing metabolites alanine, lactate and optionally bicarbonate when being MR-detected with an imaging medium containing hyperpolarized ¹³C-pyruvate. Hence it is possible to determine the grade of a tumor by comparing its metabolic profile to a known metabolic profile. When tumor progresses from an early stage primary tumor to a late stage primary tumor, the lactate signal, the total carbon signal (sum of signals of lactate plus alanine plus pyruvate and, optionally, plus bicarbonate) and - to a lesser degree - the alanine signal are significantly higher in a late stage tumor compared to an early stage tumor. Further, the corrected lactate signal, i.e. lactate to pyruvate, lactate to alanine and lactate to total carbon is also significantly higher in late stage tumors than in early stage tumors. The findings for metastases in terms of lactate signal and total carbon signal are somewhere between those observed in early and late stage tumors. Due to these characteristic findings, it is possible to determine the grade of a tumor, i.e. early stage primary tumor or a late stage primary tumor or metastases by comparing its metabolic profile with a known metabolic profile of a tumor of a certain grade, i.e. a known metabolic profile of early stage primary tumor, a known metabolic profile of late stage primary tumor or known metabolic profile of metastases.

Also embodied in this invention is a method of simultaneously identifying a tumor and determining its grade. The method is based on the method described above and a subject is examined who is suspected to have a tumor. By comparing the metabolic profile of the suspected tumor to a known metabolic profile of a tumor of a certain grade it can be determined whether the subject has a tumor at all and, if a tumor is present, the grade of said tumor.

The method of the invention can generally be used for determining the grade of a tumor. For many types of cancers an established specific grading system exists and the grade is usually rated numerically and/or descriptively. Tumors may be graded in on five-tier to two-tier scales, either rated numerically and/or descriptively, depending on the type of cancer. As an example breast cancer is traditionally graded by the Scarff-Bloom-Richardson (S-B-R) system and a pathologist using the S-B-R system looks to three structural characteristics when grading at breast tumor: nuclear pleomorphism (1), mitotic index (2), and ductoglandular differentiation (3). Tumors are graded by each criterion separately with I being the most normal (differentiated) and III being the most aberrant (undifferentiated). The scores of the three criteria are added for a final tumor grade. Therefore, the scores can range from 3-5 (well differentiated) to 6-7 (moderately differentiated) and 8-9 (poorly differentiated). As another example, prostate cancer is graded by the Gleason system as described earlier. The Gleason system identifies five levels of increasing disease aggressiveness with grade 1 being the least aggressive and grade 5 being the most aggressive cancer. To compensate for tumor heterogeneity, the two most prominent grades of the tumor are added together to produce the Gleason score.

For grading systems which are more detailed in their results than just grouping them in a two-tier (low vs. high grade tumor) or three-tier (low vs. intermediate vs. high grade tumor) grading system it is preferred to use an embodiment of the present invention which simplifies the comparison step c) and grade determination step d). A method is based on the use of a standard curve or several standard curves which correlate the signal of hyperpolarized ¹³C-pyruvate and its ¹³C-containing metabolites alanine, lactate and optionally bicarbonate with the histopathologic grade of a tumor. Said standard curve or standard curves are statistically significant standard curves which may be obtained from a patient population having a certain type of cancer.

As an example the patient population may be a patient population having prostate cancer, i.e. the patients have a tumor in the prostate and these tumors are of different grades of aggressiveness/malignancy. Said patient population undergoes MR examination of the prostate in which examination an imaging medium that comprises hyperpolarized ¹³C-pyruvate is administered and wherein subsequently MR signals of ¹³C-pyruvate and its ¹³C-containing metabolites alanine, lactate and optionally bicarbonate are detected and recorded. The tumors in this patient population are also histopathologically graded by a pathologist using the Gleason system and the two most prominent grades of the tumor are added together to produce the Gleason score. The determined Gleason score in a patient is correlated with the results of his MR examination. Various different statistical methods known in the art may be used to make this correlation. To obtain a statistically significant standard curve, said correlation is carried out for a statistically relevant number of patients, i.e. a patient population.

### DEFINITIONS

The term "grade of a tumor" in the context of the invention refers to the perceived degree of aggressiveness and/or malignancy of said tumor.

The term "MR detecting a tumor [.......] wherein signals of ¹³C-pyruvate and its ¹³C-containing metabolites alanine, lactate and optionally bicarbonate are detected" means that a signal of ¹³C-pyruvate is detected and/or a signal of its ¹³C-containing metabolite alanine is detected and/or a signal of its ¹³C-containing metabolite lactate is detected and optionally a signal of its a signal of its ¹³C-containing metabolite bicarbonate is detected. This means that either all signals, i.e. the signal of the parent compound ¹³C-pyruvate and all its ¹³C-containing metabolites are detected or only signals of a single compound, i.e. either the parent compound ¹³C-pyruvate or one of its ¹³C-containing metabolites are detected or signals of more but not all compounds, i.e. for instance ¹³C-pyruvate and ¹³C-lactate, ¹³C-pyruvate and ¹³C-alanine or ¹³C-lactate and ¹³C-alanine are detected. The above-mentioned term includes all combinations of signals of ¹³C-pyruvate and its ¹³C-containing metabolites.

The term "subject" in the context of the invention refers to any living or non-living vertebrate, preferably a living or non-living mammal like a human or non-human mammal, preferably a living mammal and most preferably a living human.

The term "hyperpolarized" is hereinafter used interchangeably with the term "polarized" and denote a nuclear polarization level in excess of 0.1%, more preferred in excess of 1% and most preferred in excess of 10%.

The term "signal" in the context of the invention refers to the MR signal amplitude or integral or peak area to noise of peaks in an MR spectrum which represent ¹³C-pyruvate and/or its ¹³C-containing metabolites alanine, lactate and optionally bicarbonate. In a preferred embodiment, the signal intensity is the peak area.

The term "total carbon signal" in the context of the invention denotes the sum of signals of ¹³C-pyruvate, ¹³C-lactate, ¹³C-alanine and optionally ¹³C-bicarbonate.

The term "metabolic profile of the tumor" in the context of the invention means the characteristic pattern of signals of ¹³C-pyruvate and/or its ¹³C-containing metabolite alanine and/or its ¹³C-containing metabolite lactate and optionally its ¹³C-containing metabolite bicarbonate. This means that in one embodiment all signals, i.e. the signal of the parent compound ¹³C-pyruvate and all its ¹³C-containing metabolites are used to generate the metabolic profile of the tumor and in another embodiment only signals of a single compound, i.e. either the parent compound ¹³C-pyruvate or one of its ¹³C-containing metabolites are used to generate the metabolic profile of the tumor. In yet another embodiment, two or more signals are used to generate the metabolic profile of the tumor, for instance the signal of the parent compound ¹³C-pyruvate and the signal of ¹³C-lactate or signals of ¹³C-pyruvate and ¹³C-alanine or signals of ¹³C-pyruvate, ¹³C-lactate and ¹³C-alanine. The above-mentioned metabolic profile may be generated using all combinations of signals of ¹³C-pyruvate and its ¹³C-containing metabolites. A "metabolic profile of the tumor" according to the invention may also include or been generated using processed signal data, e.g. ratios of signals, corrected signals, or dynamic or metabolic rate constant information deduced from the signal pattern of multiple MR detections, i.e. spectra or images.

The term "known metabolic profile of a certain tumor grade" in the context of the invention means the characteristic pattern of signals of ¹³C-pyruvate and/or its ¹³C-containing metabolites alanine, lactate and optionally bicarbonate of a tumor of a certain grade, e.g. of an early stage primary tumor (low grade tumor), of a late stage primary tumor (high grade tumor) or of metastases and wherein said characteristic pattern has been generated as described in the definition of "metabolic profile of the tumor" in the previous paragraph.

The term "stage of a tumor" in the context of the invention refers to the progression of cancer over time and either means an early stage primary tumor, a late stage primary tumor or metastases. Metastases may occur in proximity to the primary tumor, e.g. in regional lymph nodes or at sites further away from the primary tumor, e.g. in non-regional lymph nodes, bones or other sites.

The term "histopathologic grade of tumor" refers to the grade of a tumor given by a pathologist after microscopically examining a portion of the tumor, e.g. obtained from a tumor biopsy.

The term "standard curves of signal" refers to a statistically significant standard curve of signals of ¹³C-pyruvate and/or its ¹³C-containing metabolites alanine, lactate and optionally bicarbonate versus grade of a tumor. It is obtained by correlation of the signal of ¹³C-pyruvate and/or its ¹³C-containing metabolites alanine, lactate and optionally bicarbonate with the histopathologic grade of a tumor. Although written in the singular form, it can relate to a single standard curve or to one or more standard curves.

The term "certain tumor grade" refers to a tumor whose grade is known.

The terms "hyperpolarized ¹³C-pyruvate", "¹³C-pyruvate" and "pyruvate" are hereinafter used interchangeably, unless specified otherwise. In the same way, the terms "hyperpolarized ¹³C-lactate", "¹³C-lactate" and "lactate", the terms "hyperpolarized ¹³C-alanine", "¹³C-alanine" and "alanine" and the terms "hyperpolarized ¹³C-bicarbonate", "¹³C-bicarbonate" and "bicarbonate" are hereinafter used interchangeably, unless specified otherwise.

### DETAILED DESCRIPTION OF THE INVENTION

As mentioned earlier, the invention is defined in the independent claim. In step a), a tumor in a subject, preferably a living mammal, more preferably a living human or non-human mammalian animal, most preferably a living human is detected by MR.

In one embodiment, said MR detection is MR imaging or MR spectroscopy or combined MR imaging and MR spectroscopy, i.e. MR spectroscopic imaging. In another embodiment, said MR detection is MR spectroscopic imaging at various time points to determine the rate of change of parent compound ¹³C-pyruvate and/or its ¹³C-containing metabolites. In yet another embodiment, said MR detection is devised to deduce metabolic rate constants, dynamics or other characteristics of tumor progression. In a preferred embodiment, the spectral domain integrals of the resolved signals from lactate, pyruvate and alanine are obtained for each spatial point in a ¹³C-MR spectroscopic image. In another preferred embodiment, intensities from images of specific ¹³C-containing metabolites are used to generate the metabolic profile in step b) of the method of the invention.

In the MR detection of the invention, an imaging medium that comprises hyperpolarized ¹³C-pyruvate is used.

The isotopic enrichment of the hyperpolarized ¹³C-pyruvate in said imaging medium used in the method of the invention is preferably at least 75%, more preferably at least 80% and especially preferably at least 90%, an isotopic enrichment of over 90% being most preferred. Ideally, the enrichment is 100%. ¹³C-pyruvate in said imaging medium used in the method of the invention may be isotopically enriched at the C1-position (in the following denoted ¹³C₁-pyruvate), at the C2-position (in the following denoted ¹³C₂-pyruvate), at the C3-position (in the following denoted ¹³C₃-pyruvate), at the C1- and the C2-position (in the following denoted ¹³C₁,₂-pyruvate), at the C1- and the C3-position (in the following denoted ¹³C₁,₃-pyruvate), at the C2- and the C3-position (in the following denoted ¹³C₂,₃-pyruvate) or at the C1-, C2- and C3-position (in the following denoted ¹³C₁,_{2,3}-pyruvate). Isotopic enrichment at the C1-position is preferred since ¹³C₁-pyruvate has a higher T₁ relaxation in human full blood at 37° C (about 42 s) than ¹³C-pyruvate which is isotopically enriched at other C-positions.

Isotopically enriched ¹³C-pyruvate is commercially available, e.g. as sodium ¹³C-pyruvate. Alternatively, it may be synthesized as described by S. Anker, J. Biol. Chem. 176, 1948, 133-1335.

Several hyperpolarizing techniques are disclosed in WO-A-99/35508, and one of them is the dynamic nuclear polarization (DNP) technique which is preferred for the hyperpolarization of ¹³C-pyruvate. In DNP, polarization of MR active nuclei in a compound to be polarized, i.e. a sample, is affected by a polarization agent or so-called DNP agent, a compound comprising unpaired electrons. During the DNP process, energy, normally in the form of microwave radiation, is provided, which will initially excite the DNP agent. Upon decay to the ground state, there is a transfer of polarization from the unpaired electron of the DNP agent to the NMR active nuclei of the sample. Generally, a moderate or high magnetic field and a very low temperature are used in the DNP process, e.g. by carrying out the DNP process in liquid helium and a magnetic field of about 1 T or above. Alternatively, a moderate magnetic field and any temperature at which sufficient polarization enhancement is achieved may be employed. The DNP technique is for example further described in WO-A-98/58272 and in WO-A-01/96895. To polarize a sample by the DNP method, a mixture of said sample, i.e. the compound to be polarized and a DNP agent is prepared which is then frozen and inserted into a DNP polarizer for polarization. After the polarization, the mixture containing the hyperpolarized sample is rapidly transferred into the liquid state either by melting it or by dissolving it in a suitable dissolution medium. Dissolution is preferred and the dissolution process of a frozen mixture containing a DNP polarized compound and suitable devices therefore are described in detail in WO-A-02/37132. The melting process and suitable devices for the melting are for instance described in WO-A-02/36005.

In order to obtain a highly polarized sample, the sample and the DNP agent need to be in intimate contact during the DNP process. This is not the case if the mixture containing the sample and the DNP agent crystallizes upon being frozen or cooled. To avoid crystallization, either glass formers need to be present in said mixture or compounds need to be chosen for polarization which do not crystallize upon being frozen but rather form a glass.

To obtain hyperpolarized ¹³C-pyruvate by the DNP method, ¹³C-pyruvic acid or ¹³C-pyruvate is suitably used as a starting material.

Several methods for the synthesis of ¹³C₁-pyruvic acid are known in the art. Briefly, Seebach et al., Journal of Organic Chemistry 40(2), 1975, 231-237 describe a synthetic route that relies on the protection and activation of a carbonyl-containing starting material as an S,S-acetal, e.g. 1,3-dithian or 2-methyl-1,3-dithian. The dithian is metallated and reacted with a methyl-containing compound and/or ¹³CO₂. By using the appropriate isotopically enriched ¹³C-component as outlined in this reference, it is possible to obtain ¹³C₁-pyruvate, ¹³C₂-pyruvate or ¹³C_{1,2}-pyruvate. The carbonyl function is subsequently liberated by use of conventional methods described in the literature. A different synthetic route starts from acetic acid, which is first converted into acetyl bromide and then reacted with Cu¹³CN. The nitril obtained is converted into pyruvic acid via the amide (see for instance S.H. Anker et al., J. Biol. Chem. 176 (1948), 1333 or J. E. Thirkettle, Chem Commun. (1997), 1025). Further, ¹³C-pyruvic acid may be obtained by protonating commercially available sodium ¹³C-pyruvate, e.g. by the method described in US patent 6,232,497 or by the method described in WO-A-2006/038811. ¹³C-pyruvate is a commercially available compound.

The hyperpolarization of ¹³C-pyruvic acid by DNP is described in detail in WO-A1-2006/011 809. Briefly, ¹³C-pyruvic acid may be directly used for DNP since it forms a glass when frozen. After DNP, the frozen hyperpolarized ¹³C-pyruvic acid needs to be liquefied, e.g. dissolved and neutralized, i.e. converted to ¹³C-pyruvate. For the conversion, a strong base is needed. Further, since ¹³C-pyruvic acid is a strong acid, a DNP agent needs to be chosen which is stable in this strong acid.

Alternatively, ¹³C-pyruvate, i.e. a salt of ¹³C-pyruvic acid can be used for DNP. Preferred salts are those ¹³C-pyruvates which comprise an inorganic cation from the group consisting of NH₄⁺, K⁺, Rb⁺, Cs⁺, Ca²⁺, Sr²⁺ and Ba²⁺, preferably NH₄⁺, K⁺, Rb⁺ or Cs⁺, more preferably K⁺, Rb⁺, Cs⁺ and most preferably Cs⁺, as in detail described in WO-A2-2007/111515. The synthesis of these preferred ¹³C-pyruvates is disclosed in WO-A2-2007/111515 as well.

Further preferred salts are ¹³C-pyruvates of an organic amine or amino compound, preferably TRIS-¹³C₁-pyruvate or meglumine-¹³C₁-pyruvate, as in detail described in WO-A2-2007/069909. The synthesis of these preferred ¹³C-pyruvates is disclosed in WO-A2-2007/069909 as well.

The level of hyperpolarization (nuclear polarization) may for instance be determined by solid state NMR measurements of the NMR active nucleus in a solid, e.g. frozen hyperpolarized sample. For ¹³C-pyruvate, the NMR active nucleus in the hyperpolarized sample is ¹³C, and thus a solid state ¹³C-NMR of ¹³C-pyruvate or ¹³C-pyruvic acid may be acquired. The solid state ¹³C-NMR measurement preferably consists of a simple pulse-acquire NMR sequence using a low flip angle. The signal intensity of the hyperpolarized sample in the NMR spectrum is compared with signal intensity of the sample in a NMR spectrum acquired before its hyperpolarization. The level of polarization is then calculated from the ratio of the signal intensities of before and after hyperpolarization.

In a similar way, the level of polarization for a hyperpolarized sample in the liquid state (either dissolved or being a liquid at the given temperature) may be determined by liquid state NMR measurements of the NMR active nucleus in the liquid hyperpolarized sample. Again the signal intensity of the dissolved hyperpolarized sample is compared with the signal intensity of the dissolved sample before its hyperpolarization. The level of polarization is then calculated from the ratio of the signal intensities of sample before and after hyperpolarization.

The imaging medium comprising hyperpolarized ¹³C-pyruvate which is used in the method of the invention is preferably formulated as described in WO-A1-2006/011809 which also discloses suitable administration protocols and dosages.

At less than 400s after the administration of the imaging medium, preferably less than 120 s, more preferably less than 60 s after the administration, especially preferably 20 to 50 s after the administration and most preferably 30 to 40 s after the administration, an MR imaging sequence is applied that encodes the volume of interest, i.e. the tumor, in a combined frequency and spatial selective way and direct ¹³C-MR images and/or MR spectra of said volume of interest are acquired to obtain the signal intensities of ¹³C-pyruvate and its metabolites, ¹³C-lactate, ¹³C-alanine and optionally ¹³C-bicarbonate.

In a preferred embodiment, dedicated radiofrequency coils, preferably dedicated ¹³C-coils are used for the MR detection. The type of coils used is of course dependent on the location of the tumor/type of cancer. As an example a bird cage coil may be used for MR detection of tumors in the brain and an endorectal coil may be used for MR detection of tumors in the prostate.

In step b) of the method of the invention, the above-mentioned signals of ¹³C-pyruvate and its metabolites, ¹³C-lactate, ¹³C-alanine and optionally ¹³C-bicarbonate and optionally the total carbon signal are used to generate a metabolic profile of the tumor.

In one embodiment, the spectral signal intensities of ¹³C-pyruvate and its metabolites, ¹³C-lactate, ¹³C-alanine and optionally ¹³C-bicarbonate (hereinafter denoted "¹³C-pyruvate and its metabolites") are used to generate a metabolic profile of the tumor. In another embodiment, the spectral signal integrals of ¹³C-Pyruvate and its metabolites are used to generate a metabolic profile of the tumor. In another embodiment, signal intensities from separate images of ¹³C-pyruvate and its metabolites are used to generate a metabolic profile of the tumor. In yet another embodiment, the signal intensities are obtained at two or more time points to calculate the rate of change of ¹³C-pyruvate and/or its metabolites.

In one embodiment, all signals are used to generate a metabolic profile of the tumor, i.e. the signal of ¹³C-pyruvate and ¹³C-lactate and ¹³C-alanine and optionally ¹³C-bicarbonate. Further, the total carbon signal, i.e. sum of signals of ¹³C-lactate, ¹³C-alanine, ¹³C-pyruvate and, optionally, ¹³C-bicarbonate may be used to generate the metabolic profile of the tumor. In another embodiment, one or more selected signals are used to generate the metabolic profile of the tumor. Thus in a preferred embodiment, the lactate signal and/or the alanine signal and/or the total carbon signal is used to generate the metabolic profile of the tumor. In a further preferred embodiment, the lactate signal and/or the total carbon signal is used to generate the metabolic profile of the tumor.

In another embodiment, the metabolic profile of the tumor includes or is generated using processed signals data, e.g. ratios of signals, corrected signals, or dynamic or metabolic rate constant information deduced from the signal pattern of multiple MR detections, i.e. spectra or images. Thus, in a preferred embodiment a corrected lactate signal, i.e. lactate to alanine signal and/or lactate to pyruvate signal and/or lactate to total carbon signal is used to generate the metabolic profile of the tumor. In another preferred embodiment, the processed signal results in a number for each image point that is characteristic of the metabolic profile of said each image point.

In step c) of the method of the invention the metabolic profile of the tumor obtained in step b) is compared to a known metabolic profile of a tumor of a certain grade.

A know profile of a tumor of a certain grade is preferably based on MR examination of a statistically significant number of patients having a certain type of cancer. As an example a statistically significant number of patients having prostate cancer, i.e. patients having a tumor in the prostate are used to generate a known profile of a prostate tumor of a certain grade. In one embodiment, an imaging medium that comprises hyperpolarized ¹³C-pyruvate is administered to the patients and subsequently MR signals of ¹³C-pyruvate and its ¹³C-containing metabolites alanine, lactate and optionally bicarbonate are detected and recorded. These signals and optionally the total carbon signal are used to generate a metabolic profile of each patient's tumor in the way described in the previous paragraphs. The tumors in these patients are also histopathologically graded by a pathologist using the Gleason system and the two most prominent grades of the tumor are added together to produce the Gleason score. Metabolic profiles of patients having the same Gleason score are pooled and an "average" metabolic profile is generated by statistical methods known in the art of these pooled profiles which correlate with a given Gleason score. Such an "average" metabolic profile is a known metabolic profile of a tumor of a certain grade.

In one embodiment the known metabolic profile contains all signals, i.e. the signal of ¹³C-pyruvate and ¹³C-lactate and ¹³C-alanine and optionally ¹³C-bicarbonate. Further, it may contain the total carbon signal, i.e. sum of signals of lactate, alanine, pyruvate and, optionally, bicarbonate. In another embodiment, the known metabolic profile contains one or more selected signals. Thus in a preferred embodiment, the lactate signal and/or the alanine signal and/or the total carbon signal are present in said known metabolic profile. In a further preferred embodiment, the lactate signal and/or the total carbon signal are present in said known metabolic profile.

In another further preferred embodiment, the known metabolic profile contains processed signals data, e.g. ratios of signals, corrected signals, or dynamic or metabolic rate constant information deduced from the signal pattern of multiple MR detections, i.e. spectra or images. Thus, in a preferred embodiment the known metabolic profile contains a corrected lactate signal, i.e. lactate to alanine signal and/or lactate to pyruvate signal and/or lactate to total carbon signal In another preferred embodiment, the processed signal results in a number for each image point that is characteristic of the metabolic profile of said each image point.

The known metabolic profile may be in the form of a table, i.e. displaying signal values for one or more of the signals described above. In another embodiment, such a known profile may be in the form of a graphic plot. In yet another embodiment such a known profile may be in the form of a data set and said data set is stored on a computer readable medium.

In its simplest embodiment, the comparison can be done by a visual comparison of the metabolic profile of the tumor generated in step b) of the method of the invention and the known metabolic profile of a tumor of a certain grade. In another embodiment the comparison is done in an automated fashion, e.g. by using a suitable software which compares the two metabolic profiles. In one embodiment the metabolic profile of the tumor generated in step b) is compared to a single known metabolic profile of a tumor of a certain grade. As an example, the metabolic profile of the tumor, for instance a prostate tumor, generated in step b) is compared to a single known metabolic profile of prostate tumors having a Gleason score 4 (2+2). If the metabolic profile of the prostate tumor under examination and the known metabolic profile of prostates tumor with the Gleason score 4 (2+2) is highly similar, there will be no immediate need to compare said metabolic profile to other known metabolic profiles of prostate tumors with other Gleason scores, for instance a Gleason score 8 (4+4). However, usually metabolic profile of the tumor generated in step b) is compared to several known metabolic profile of a tumor of a several certain grades. In a preferred embodiment, the metabolic profile obtained in step b) is displayed as a parametric image overlay on an anatomic image, using a color and/or numeric correlated with the tumor grade. By doing so the image overlay obtained provides combined information on tumor grade and tumor stage, i.e. extent and location of the tumor. In another preferred embodiment the tumor area is defined in an anatomic image, e.g. a proton anatomic image, and this tumor area with its metabolic profile obtained in step b) then forms the basis for an automated calculation of a tumor grade. As such the grading relies on an automated look-up of the grade in a table, database and the like.

In step d) of the method of the invention the grade of the tumor under examination is determined based on the similarities and differences between the metabolic profile of the tumor and the known metabolic profile of a tumor of a certain grade. In a simplified way, the higher the similarity between known metabolic profile and metabolic profile of the tumor under examination is, the higher the likelihood that the tumor under examination has the same grade as the tumors the known metabolic profile is based on. It is apparent that there are statistical methods which are well known in the art to evaluate and calculate the degree of similarity or difference between tumor metabolic profile and known metabolic profiles of a tumor of a certain grade and the use of such statistical methods is preferred.

As mentioned earlier, the method above is based on the use of a standard curve or several standard curves which correlate the signal of ¹³C-pyruvate and its ¹³C-containing metabolites with the histopathologic grade of a tumor. Said standard curve or standard curves are statistically significant standard curves which may be obtained from a patient population having a certain type of cancer in a way as described earlier in this application.

The method above is especially suitable for cancer types with established grading systems which are more detailed in their results than just grouping those results in a two-tier (low vs. high grade tumor) or three-tier (low vs. intermediate vs. high grade tumor) grading system. In a preferred embodiment the method above is used for grading of prostate cancer, i.e. grading of tumors in the prostate.

Thus, in one embodiment, the spectral signal intensities of ¹³C-pyruvate and its metabolites, ¹³C-lactate, ¹³C-alanine and optionally ¹³C-bicarbonate (hereinafter denoted "¹³C-pyruvate and its metabolites") are used to determine the grade of the tumor from a standard curve of such signals versus tumor grade. In another embodiment, the spectral signal integrals of ¹³C-pyruvate and its metabolites are used. In another embodiment signal intensities from separate images of ¹³C-pyruvate and its metabolites are used. In yet another embodiment, the signal intensities are obtained at two or more time points to calculate the rate of change of ¹³C-pyruvate and/or its metabolites.

In one embodiment, all signals are used to determine the grade of the tumor from a standard curve of such signals versus tumor grade, i.e. the signal of ¹³C-pyruvate and ¹³C-lactate and ¹³C-alanine and optionally ¹³C-bicarbonate. Further, the total carbon signal, i.e. sum of signals of lactate, alanine, pyruvate and, optionally, bicarbonate may be used. In another embodiment, one or more selected signals are used to determine the grade of the tumor from a standard curve of such signals versus tumor grade. Thus in a preferred embodiment, the lactate signal and/or the alanine signal and/or the total carbon signal is used. In a further preferred embodiment, the lactate signal and/or the total carbon signal is used.

In another embodiment, processed signal data, e.g. ratios of signals, corrected signals, or dynamic or metabolic rate constant information deduced from the signal pattern of multiple MR detections, i.e. spectra or images are used to determine the grade of the tumor from a standard curve of such signals versus tumor grade. Thus, in a preferred embodiment a corrected lactate signal, i.e. lactate to alanine signal and/or lactate to pyruvate signal and/or lactate to total carbon signal is used to determine the grade of the tumor from a standard curve of such signals versus tumor grade. In another preferred embodiment, the processed signal results in a number for each image point that is characteristic of the metabolic profile of said each image point.

In its simplest embodiment, a standard curve may be a linear correlation between signal and histopathologic tumor grade or score, e.g. for the Gleason grading system. In such an embodiment, the determination of step b) can be done "manually" by using the detected signals or data obtained from processing these signals and comparing them with the corresponding signals in the standard curve which form one axis of said standard curve. By using the standard curve, the corresponding tumor grade or score can be found on the other axis.

In another embodiment, it is anticipated that the standard curve is in the form of an algorithm which performs the determination of the tumor grade or tumor score based on the signals detected in step a) in an automated way. The algorithm may further contain other statistical analytics which allow for the evaluation of the confidence level of the tumor grade determination.

### DESCRIPTION OF FIGURES

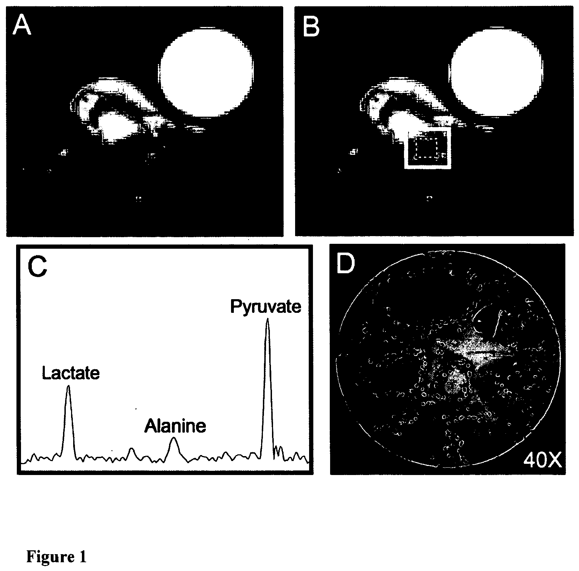

**Figure 1** shows representative T₂ weighted axial images (A), T₂ weighted images with a overlaid grid showing the 0.135 cm³ hyperpolarized ¹³C spectra corresponding to the selected pathology (B), the corresponding spectra (C) and H&E (hematoxylin and eosin) stained histologic section (D) taken from a normal mouse prostate.

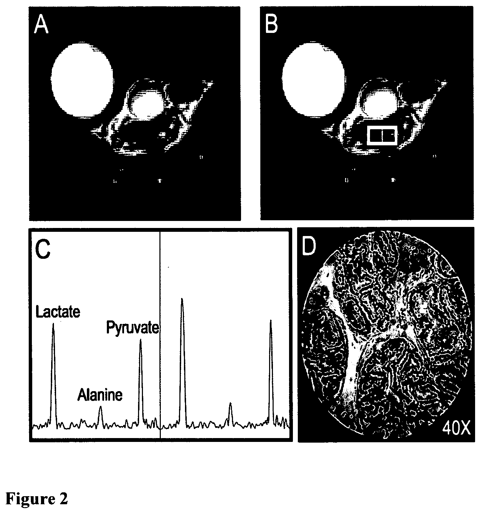

**Figure 2** shows representative T₂ weighted axial images (A), T₂ weighted images with a overlaid grid showing the 0.135 cm³ hyperpolarized ¹³C spectra corresponding to the selected pathology (B), the corresponding spectra (C) and H&E stained histologic section (D) taken from a TRAMP mouse prostate with an early stage primary tumor (low grade tumor).

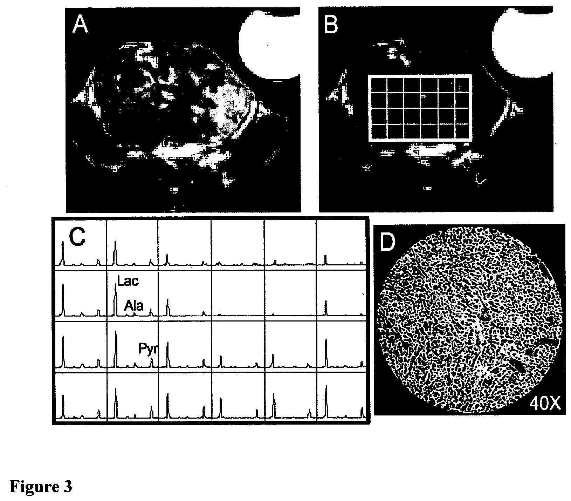

**Figure 3** shows representative T₂ weighted axial images (A), T₂ weighted images with a overlaid grid showing the 0.135 cm³ hyperpolarized ¹³C spectra corresponding to the selected pathology (B), the corresponding spectra (C) and H&E stained histologic section (D) taken from a TRAMP mouse prostate with a late stage primary tumor (high grade tumor).

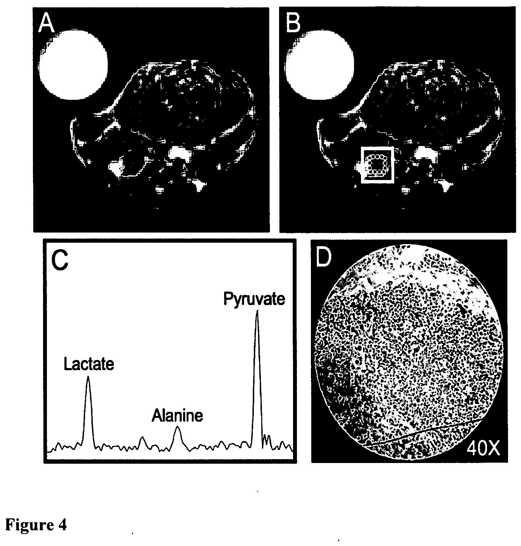

**Figure 4** shows representative T₂ weighted axial images (A), T₂ weighted images with a overlaid grid showing the 0.135 cm³ hyperpolarized ¹³C spectra corresponding to the selected pathology (B), the corresponding spectra (C) and H&E stained histologic section (D) taken from a TRAMP mouse prostate with a late stage primary tumor (high grade tumor) and a region of lymph node metastasis.

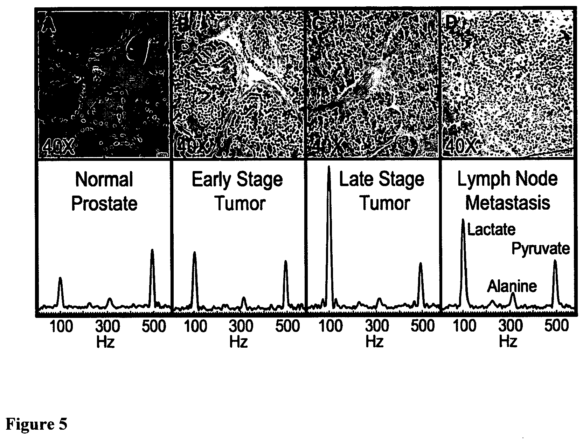

**Figure 5** summarizes the metabolic and histological changes that occurred with the evolution and progression of prostate tumor in the TRAMP model and compares these to the metabolic profile and histology of normal murine prostate tissue.

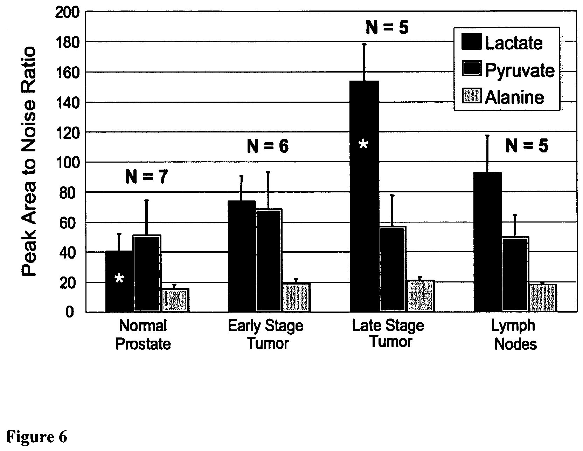

**Figure 6** shows a bar plot of hyperpolarized ¹³C₁-pyruvate to noise ratios and its ¹³C-containing metabolites alanine and lactate to noise ratios of normal murine prostate, TRAMP mouse early stage prostate tumor, TRAMP mouse late stage prostate tumor, and lymph node metastases.

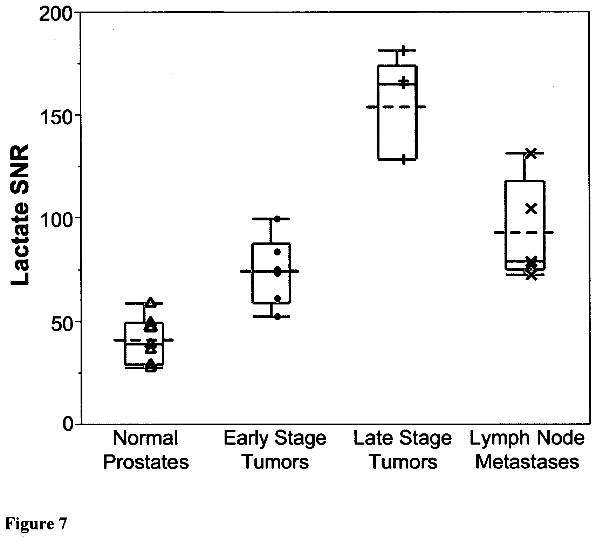

**Figure 7** shows a box plot of lactate signal to noise ratio versus tissue type, i.e. normal murine prostate tissue, TRAMP mouse early stage prostate tumor tissue, TRAMP mouse late stage prostate tumor tissue, and lymph node metastases.

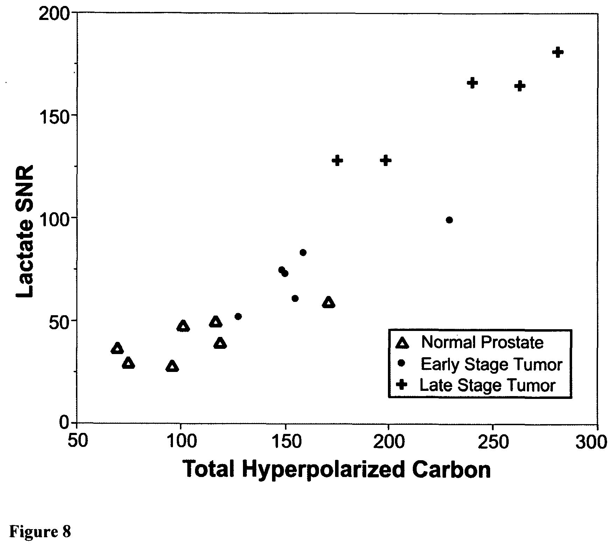

**Figure 8** shows a plot of lactate signal to noise ratio versus total hyperpolarized carbon for various tissue types, i.e. normal murine prostate tissue, TRAMP mouse early stage prostate tumor tissue and TRAMP mouse late stage prostate tumor tissue.

### Examples

The Transgenic Adenocarcinoma of Mouse Prostate (TRAMP) model is particularly useful for studying the metabolic changes that occur with prostate cancer evolution and progression since TRAMP mice demonstrate histopathologic disease progression and associated metabolic changes that mimic the human disease. The following study shows the quantification of changes in hyperpolarized ¹³C₁-pyruvate metabolism with prostate cancer progression in TRAMP mice using histopathology of the resected malignant tissue as the standard of reference.

### MR detection

All normal (N=5) and TRAMP (N=4 early stage primary tumor (low grade tumor)), N=5 late stage primary tumor (high grade tumor)) mouse studies were performed under a protocol approved by the UCSF Institutional Animal Care and Utilization Committee. (1). For all the mice included in this study, a 28 g port that extended from the dorsal surface of their neck was surgically implanted into one of their jugular veins at least one day before the hyperpolarized MR study. At the time of the MR experiment, the mice were anesthetized with 1-1.5% isoflurane and a 90 cm long, 24 g extension tube was connected to the jugular vein port using a hard plastic connector (Strategic Applications, Libertyville, IL). This special extension tubing made it possible to quickly inject the hyperpolarized agent inside of human MR scanner while maintaining a low dead volume in the tubing. Next, the mice were placed on a water filled, temperature controlled pad that was heated to approximately 37° C by means of circulating water and positioned inside of custom built dual-tuned proton-carbon T/R coil. The coil design consisted of a quadrature ¹³C coil inside of ¹H quadrature coil with an inner diameter of 5 cm, a length of 8 cm, and open slots on the top surface to allow visual monitoring of the mouse while inside the coil. The entire setup was placed on the patient table and positioned in the bore of a 3 T human GE scanner equipped with the multinuclear spectroscopy package.

¹H-MR images were acquired in sagittal, axial and coronal views using a T₂-weighted fast spin echo sequence with a 6 cm FOV, 128 x 128 matrix, 1.5 mm slice thickness with no inter-slice spacing, and TE = 102 ms/TR = 4 s. Once the ¹³C₁-pyruvate was hyperpolarized (21.7 ± 2.0%) and dissolved (7.9 ± 0.2) as described in WO-A-2006/011809, the solution was quickly transported to the MR scanner and 350 ± 50 µL of 79 mM hyperpolarized pyruvate was injected into the mouse across a 12 second interval. The injection was immediately followed by a 150 µL saline flush to clear the hyperpolarized pyruvate from the jugular vein catheter and tubing. A double spin-echo pulse sequence with a small flip-angle excitation, adiabatic refocusing, and a flyback echo-planar readout trajectory was used to acquire *in vivo* 3D hyperpolarized ¹³C-MRSI data 35 seconds after the start of the pyruvate injection into jugular vein of six TRAMP mice (3 early and 3 late stage tumor) (1-2). 3D-MRSI data were acquired from the abdomen of the mouse using a TE/TR of 140/215, an 8x8x16 matrix, a 40mmx40mmx86.4mm FOV (0.135 cm³ resolution) and an acquisition time of 14 seconds.

To correlate pathology with proton images of the TRAMP mice, sagittal, coronal and axial images were displayed on a laptop computer during dissection. During the dissection digital photographs and videos were taken and excised tissues were immediately fixed in 10% buffered formalin. Fixed tissue specimens were subsequently moved into 70% ethanol and processed into paraffin blocks. Tissue blocks were sectioned on a Leica microtome at 5 micron thickness. Sections were dried onto glass slides and stained using a standard hemotoxylin and eosin protocol. The % of normal, well differentiated (WD), moderately well differentiated (MWD) and poorly differentiated (PD) was used to classify tumor's into early (low grade) and late stage (high grade) categories (Table 1). The % necrosis was also recorded (Table 1).

**Table 1**

| | | **Histological grading % of prostate/tumor or LN by grade** | | | | |
|---|---|---|---|---|---|---|
| **TRAMP** | **Gross Tumor Grading** | | | | | |
| --- | --- | Normal | WD | MWD | PD | |
| MS36 | early stage low grade tumor | 1 | 33 | 1 | 65 | |
| MS44 | early stage low grade tumor | 1 | 94 | 5 | 0 | |
| MS54 | early stage low grade tumor | 2 | 35 | 35 | 30 | |
| MS72 | early stage low grade tumor | 15 | 78 | 7 | 0 | |
| MS51 | late stage high grade tumor | 0 | 0 | 1 | 99 | |
| MS53 | late stage high grade tumor | 0 | 0 | 0 | 100 | |
| MS61 | late stage high grade tumor | 0 | 0 | 0 | 100 | **% Necrotic** |
| | | | | | | |
| LN-MS51R | late stage high grade tumor, lymph node metastases | 2 | | | 98 | -> 20 |
| LN-MS51L | late stage high grade tumor, lymph node metastases | 0 | | | 100 | -> 50 |
| LN-MS53R | late stage high grade tumor, lymph node metastases | 2 | | | 98 | -> 5 |
| LN-MS61 | late stage high grade tumor, lymph node metastases | 2 | | | 98 | -> n.d |

### Data Analysis and generation of tumor metabolic profiles

MRI/¹³C MRSI data were processed and aligned using custom software. The primary and metastatic regions of prostate cancer identified on pathology were traced on the corresponding high spatial resolution anatomic images. Every voxel associated with a tissue type of interest was integrated over a predetermined frequency range for each of the metabolites. All the integrals were scaled by the noise calculated from a region of the spectrum that did not contain any metabolites. The scaled integrals for a given metabolite and tissue type were averaged together for each study resulting in one metabolic profile, i.e. set of lactate, alanine and pyruvate numbers for each study. The average values from the various studies were statistically compared using JMP to perform an Analysis of Variance on the data. If a statistical significant difference was found, the four tissue types were compared in a pair-wise manor using a t-Test with Tukey correction for multiple comparisons. In addition, a Random Effects model was incorporated into all of the statistical tests to correct for any correlation that may have been introduced into the data by including multiple studies from the same animal.

### Results

Figures 1-4 show representative T₂ weighted axial images (A), T₂ weighted images with a overlaid grid showing the 0.135 cc hyperpolarized ¹³C spectra corresponding to the selected pathology (B), the corresponding spectra (C) and H&E stained histologic section (D) taken from a normal mouse prostate (Figure 1), early stage primary tumor in a TRAMP mouse prostate (Figure 2), late stage primary tumor (Figure 3) and a region of lymph node metastasis (Figure 4). As can be seen in Figure 1, the normal murine prostate is a very small (about 0.022 cc) organ and care had to be taken to select a voxel that encompassed the murine prostate while minimizing spectral contribution from surrounding tissue. This was accomplished in post processing by voxel-shifting the zero filled spectral data (dashed box, real resolution is the solid line) until it maximally overlapped the murine prostate (1B). The corresponding hyperpolarized ¹³C spectra (1C) demonstrated resonances for pyruvate (173 ppm) and its metabolic products lactate (185 ppm) and alanine (178 ppm). The pyruvate resonance was largest followed by lactate, alanine and a small resonance for pyruvate hydrate. Similar to the healthy human prostate, the murine prostate is a glandular organ with glandular epithelial cells surrounding ducts held together by stromal tissue as shown in the H&E section (1D).

With the development of an early stage prostate tumor in the TRAMP mouse (Figure 2), the size of the prostate significantly increased allowing for two or more 0.135 cc voxels to be placed completely within the tumor (2B). In the corresponding hyperpolarized ¹³C-spectra, there was a dramatic increase in lactate to levels that were equal to or greater than pyruvate as well as an increase in the peak area to noise of the pyruvate and alanine resonances relative to the normal mouse prostate. The corresponding histopathology (2C) demonstrates the characteristic increase in number of smaller glandular cells and loss of ductal volume as well as stromal thickening associated with prostate cancer.

In later stage primary disease (Figure 3), the tumors became very large and on pathology appearing as anaplastic sheets of pleomorphic cells with irregular nuclei and a very little cytoplasm surrounding the nuclei as well as almost complete loss of ductal morphology and areas of necrosis (3D). It was possible to acquire arrays of hyperpolarized ¹³C spectra from these tumors and the spectra demonstrated very high levels of lactate with the lactate to pyruvate ratios being significantly higher than early stage (3.4 vs 1.2) disease. There was also metabolic heterogeneity in these large tumors, with variability in the lactate/pyruvate ratio as well as very reduced metabolite levels in necrotic regions (3C).

Figure 4 shows a representative case of lymph node metastases in a late stage tumor. The hyperpolarized metabolic findings for lymph node metastases in terms of hyperpolarized lactate, alanine and pyruvate levels were somewhere between those observed in early and late stage tumors. This could have been due to the large heterogeneity of lymph node metastases as compared to primary late stage tumors (Table 1).

Figure 5 summarizes the metabolic and histological changes that occurred with the evolution and progression of cancer in the TRAMP model. Figure 6 shows a bar plot of hyperpolarized metabolite to noise ratios between normal, early stage, late stage, and lymph node metastases. Early stage tumors demonstrated a significant increase in lactate and an increase in overall hyperpolarized carbon signal (sum of hyperpolarized lactate, alanine and pyruvate). When tumor progressed from an early stage primary tumor to a late stage primary tumor, the hyperpolarized lactate signal to noise, the total hyperpolarized carbon signal significantly increased again. More importantly, the box plot of lactate S/N versus tissue type in Figure 7 demonstrated minimal overlap of individual lactate values between normal, early stage and late stage tissues, although there was overlap between early stage and lymph node metastases. The best separation of normal, early and late stage tumors was obtained by plotting hyperpolarized lactate versus total hyperpolarized carbon (Figure 8). Only one normal study overlapped with early stage tumors and this normal study had both higher lactate and total hyperpolarized carbon that the other normals.

## Claims

1. Use of hyperpolarized ¹³C-pyruvate for the manufacture of an MR imaging medium used in a method of determining the grade of a tumor, said method comprising the steps of:
a) MR detecting a tumor in a subject using said MR imaging that comprises hyperpolarized ¹³C-pyruvate wherein signals of ¹³C-pyruvate and its ¹³C-containing metabolites alanine, lactate and optionally bicarbonate are detected;
b) using the lactate to total carbon signal to generate a metabolic profile of the tumor;
c) comparing the metabolic profile of the tumor generated in step b) to a known metabolic profile of a tumor of a certain grade; and
d) determining the grade of the tumor based on the similarities and differences between said metabolic profile of the tumor and said known metabolic profile.

2. Use according to claim 1, wherein said method is a method of determining the grade of a prostate tumor.

## Patentansprüche

1. Verwendung eines hyperpolarisierten ¹³C-Pyruvats zur Herstellung eines in einem Verfahren zur Bestimmung des Grades eines Tumors verwendeten MRT-Abbildungsmediums, welches Verfahren die folgenden Schritte umfasst:
a) MRT-Nachweis eines Tumors in einem Individuum unter Verwendung der MRT-Abbildung, welche ein hyperpolarisiertes ¹³C-Pyruvat umfasst, wobei Signale von ¹³C-Pyruvat und dessen ¹³C-haltigen Metaboliten Alanin, Lactat und eventuell Bicarbonat nachgewiesen werden;
b) Verwendung des Lactat-zu-Gesamtkohlenstoff-Signals zur Erzeugung eines metabolischen Profils des Tumors;
c) Vergleichen des im Schritt b) erzeugten metabolischen Profils des Tumors mit einem bekannten metabolischen Profil eines Tumors eines bestimmten Grades; und
d) Bestimmung des Tumorgrades auf der Grundlage der Ähnlichkeiten und Unterschiede zwischen dem metabolischen Profil des Tumors und dem bekannten metabolischen Profil.

2. Verwendung nach Anspruch 1, wobei das Verfahren ein Verfahren zur Bestimmung des Grades eines Prostatatumors ist.

## Revendications

1. Utilisation du pyruvate ¹³C pour la fabrication d'un milieu d'imagerie RM utilisé dans un procédé de détermination de la teneur d'une tumeur, ledit procédé comprenant les étapes consistant à:
a) détecter par résonance magnétique une tumeur dans un sujet en utilisant ladite imagerie RM qui comprend du pyruvate ¹³C hyper-polarisé où les signaux du pyruvate ¹³C et de ses métabolites ¹³C contenant de l'alanine, du lactate et éventuellement du bicarbonate sont détectés;
b) utiliser le lactate au signal de carbone total pour générer un profil métabolique de la tumeur;
c) comparer le profil métabolique de la tumeur générée dans l'étape b) à un profil métabolique connu d'une tumeur d'une certaine teneur; et
d) déterminer la teneur de la tumeur en fonction des similitudes et des différences entre ledit profil métabolique de la tumeur et ledit profil métabolique connu.

2. Utilisation selon la revendication 1, dans laquelle ledit procédé est un procédé de détermination de la teneur d'une tumeur de la prostate.
